# EUROPEAN PATENT APPLICATION

(11) **EP 3 246 022 A1**
(43) Date of publication of application: **22.11.2017**
(21) Application number: 16737099.8
(22) Date of filing: 11.03.2016
(51) Int. Cl.: A61K 31/05, A61K 31/222, A61P 9/10, A61P 25/28, A61K 9/20, A61K 9/48, A61K 9/08, A61K 9/107, A61K 9/127, A61K 9/19, A61K 9/16, C07C 39/15

(54) **THE USE OF DIPHENOL IN PREPARATION OF MEDICINES FOR PREVENTION AND TREATMENT OF CEREBRAL ISCHEMIA**

(30) Priority: 13.01.2015 CN 201510016510
(71) Applicant: Xi'an Libang Pharmaceutical Co., Ltd, Xi'an, Shaanxi 710075 (CN)
(72) Inventor: WANG, Rutao, Xi'an, Shaanxi 710075 (CN); CHEN, Tao, Xi'an, Shaanxi 710075 (CN); AN, Long, Xi'an, Shaanxi 710075 (CN); ZHAO, Yi, Xi'an, Shaanxi 710075 (CN); WANG, Weijiao, Xi'an, Shaanxi 710075 (CN); GUO, Shupan, Xi'an, Shaanxi 710075 (CN); XIAO, Sa, Xi'an, Shaanxi 710075 (CN); PANG, Jinghua, Xi'an, Shaanxi 710075 (CN); HU, Huijing, Xi'an, Shaanxi 710075 (CN)
(74) Representative: Habermann, Hruschka & Schnabel
(86) International application number: PCT/CN2016/076112
(87) International publication number: WO 2016/112874

(57) **Abstract**

The present invention relates to use of biphenols in the preparation of a medicament for the prevention and treatment of ischemic stroke, specifically to use of 3,3',5,5'-tetraisopropyl-4,4'-biphenol and salt, ester, or solvate thereof in the preparation of a medicament for the prevention and treatment of ischemic stroke injury.

## Description

### Technical Field

The present invention belongs to the field of pharmaceutical technology and relates to a novel use of a drug, and specifically to a novel use of biphenol in the manufacture of a medicament for the treatment and/or prevention of ischemic stroke.

### Background

In recent years, stroke has become a common disease being serious threat to the health of humans, especially in the elderly over the age of 50, characterized by high incidence, high morbidity, high mortality, high recurrence rate, and multiple complications, i.e. "four-highs and one-multi". In stroke patients, intracerebral arterial stenosis, occlusion or rupture are caused by various predisposing factors, resulting in acute cerebral blood circulation disorders, clinically manifested as a transient or permanent brain dysfunction symptoms and signs.

Statistically, stroke leads to death of 40 million people over the world each year, with an annual incidence of 2 million just in China. Among the 7 million surviving patients, 4.5 million patients lose labor capability to varying degrees as well as self-care capability. The disability rate is as high as 75%. In China, 1.2 million patients die from stroke each year. Those patients who had stroke are prone to have a relapse, and the situation will become worse with each relapse. Therefore, effective means to prevent stroke recurrence are greatly needed.

Ischemic stroke accounts for about 80% of all stroke conditions, which is softening necrosis of local brain tissues due to blood circulation disorders, ischemia, and hypoxia. Its onset is mainly due to atherosclerosis and thrombosis occurring in the arteries that supply blood to the brain, causing stenosis or even occlusion, resulting in focal acute cerebral blood supply insufficiency. Also, foreign objects (solid, liquid, or gas) entering from the blood circulation into the cerebral arteries or the neck arteries that supply to the cerebral blood circulation cause blood flow obstruction or sudden decrease in blood flow volume and consequently brain tissue softening necrosis in the corresponding dominating area.

There are two major causes of ischemic brain injury: (1) due to insufficient productivity after ischemia, ATP-dependent enzyme activity and physiological activities are suppressed, chloride ions, sodium ions and water flow cause cell edema, and synaptic interstitial excitatory amino acids (mainly glutamate) accumulate, resulting in excessive activation of glutamate receptors; with increase in calcium influx mediated by NMDA and other receptors, cell depolarization due to potassium efflux, and opening of voltage-sensitive calcium channels, intracellular calcium overloads and a variety of enzymes including phospholipase and nitric oxide synthase (NOS) are excessively activated, thereby generating a series of active metabolites and free radicals and consequently causing cell damage; (2) ischemic tissues in stroke patients after being treated acquire blood perfusion or spontaneous reperfusion which inevitably lead to cerebral ischemia-reperfusion injury, despite of the regaining of nutrients; in other words, although blood supply is restored at a certain time after cerebral ischemia, not only the function thereof fails to recover, but signs of more serious brain dysfunction appear.

Ischemic brain injury involves very complex pathophysiological processes, in which the interactions between the various aspects and various factors have not been fully elucidated. Nevertheless, currently the following mechanisms are considered to play an essential role in ischemic brain injury:

### (1) excitatory amino acid toxicity and ischemic brain damage

A large number of studies have shown that increased excitotoxicity of excitatory amino acid (EAA) during ischemia played an important role in ischemic nerve cell injury. Excitatory amino acids mainly refer to glutamate (Glu) and aspartate (Asp). The postsynaptic neurons overexcited EAA may activate intracellular signal transduction pathways, allowing some receptors to amplify the second messenger effect caused by normal physiological stimuli and triggering the expression of proinflammatory genes after ischemia. Excitatory amino acids such as Glu and Asp play a key role in ischemic nerve cell injury. The longer the ischemic duration, the higher the peak concentration of Glu and Asp in brain interstitial tissues, and the more severe the neuropathological and neurological damages, which is consistent with EAA toxicity being concentration-dependent. The toxic effects of excitatory amino acids on nerve cells are shown in various aspects: excessive EAA activates its receptors, resulting in continuous depolarization of excitatory neurons, which in turn causes intracellular Ca²⁺ overload and consequently lead to cell necrosis; increase in free radical (such as nitric oxide) production is promoted, and cytotoxicity is induced by the free radicals; EAA participates in a variety of metabolic processes in the brain, blocking the tricarboxylic acid cycle and decreasing ATP production, leading to increased cell toxicity by EAA.

### (2) free radicals and lipid peroxidation and ischemic brain damage

Ischemic brain injury is a complex pathophysiological process involving multiple factors. Generally, it is considered to be associated with tissue lipid peroxidation caused by oxygen free radicals and irreversible damage caused by intracellular calcium overload. Its detrimental effects can be summarized as: acting on polyunsaturated fatty acids, and leading to lipid peroxidation; inducing cross-linking of macromolecules such as DNA, RNA, polysaccharides, and amino acids, with the original activity or function of the cross-linked macromolecules being lost or attenuated; promoting the polymerization and degradation of polysaccharide molecules; free radicals widely attacking unsaturated fatty acid-rich nerve membranes and blood vessels, inducing a lipid peroxidation waterfall effect, resulting in protein denaturation, breaking of polynucleotide strands, and base re-modification, causing damage to cell structure integrity, and seriously affecting membrane permeability, ion transportation, and membrane barrier function, thereby leading to cell death. Free radicals also evoke an increase in EAA release, leading to reperfusion injury after cerebral ischemia.

### (3) Ca²⁺ overload and cerebral ischemic brain injury

Ca²⁺ overload in ischemic brain injury is a result of the combined effects of various factors, and is a common pathway for the action of various factors in the process of cerebral ischemic injury. The impact of Ca²⁺ in ischemic brain injury mainly includes:
a) Mitochondrial dysfunction: when the intracellular and extracellular calcium balance is disrupted, extracellular Ca²⁺ flows into cells and mainly accumulate in mitochondria, and Ca²⁺ may inhibit ATP synthesis, impeding energy generation. Ca²⁺ activates phospholipases on mitochondria, causing mitochondrial membrane damage. In addition to ATP synthesis, mitochondria play an important role in cellular redox reactions and maintenance of osmotic pressure, pH value, and cytoplasmic signals, and mitochondria is the important target of cell damage.
b) Enzyme activation: Ca²⁺ activates Ca²⁺-dependent phospholipases (mainly phospholipase C and phospholipase A2) and promote membrane phospholipid degradation; the free fatty acids, prostaglandins, leukotrienes, lysophospholipids and the like that are produced in the process of membrane phospholipid degradation are toxic to cells; Ca²⁺ also activates calcium-dependent proteases and converts the intracellular non-toxic xanthine dehydrogenase into xanthine oxidase, with large amounts of oxygen free radicals generated; Ca²⁺ may activate NOS.

It has been demonstrated in experiments that the above pathophysiological changes could somehow be intervened by drugs. Compared to patients with drug withdrawal, those with long-term use of reliable drugs for prevention and treatment of ischemic stroke have their recurrence rate reduced by 80% or more and mortality reduced by 90% or more. Among patients who have taken medication for a long time over three years, 80% or more is not at risk of recurrence, and very few shows slight recurrence. This has provided a theoretical foundation for medicinally combating ischemic brain injury. Currently, commonly used drugs against cerebrovascular diseases mainly include the following categories:
GABA receptor agonists: GABA can antagonize the excitotoxicity of excitatory amino acids, and functions in inhibition protection; a representative drug is clomethiazine;
NMDA receptor antagonists: antagonizing NMDA receptors, thereby inhibiting calcium influx mediated by them; a representative drug is MK801;
Calcium ion antagonists: preventing intracellular calcium overload, preventing vasospasm, and increasing blood flow; a representative drug is nimodipine;
Anti-free radicals drugs: scavenging free radicals, inhibiting lipid peroxidation, thereby inhibiting oxidative damage to brain cells, vascular endothelial cells and nerve cells; a representative drug is edaravone.

However, the specific mechanism of ischemic stroke has not been clarified and is considered to be a very complex pathophysiological process with interaction of many factors; whereas, the above drugs act by simple mechanisms, with uncertain clinical therapeutic effects or serious side effects, so that their application in the treatment of ischemic stroke is limited.

In recent years, many domestic and foreign studies have found that the anesthetic propofol may have a very positive impact on ischemic stroke. In animal and *in vitro* experiments, and even in some clinical studies, propofol has been proven to have significant protective and therapeutic effects on neurological impairment. It has been demonstrated in the experiments that propofol could not only block the sodium ion flow or reduce Glu release activated by potassium ions by activating the GABA receptor, but also block the inhibition of Glu transportation by glial cells after oxidative treatment, both eventually reducing extracellular Glu concentration, delaying or preventing excitatory neuron death; propofol could inhibit extracellular calcium influx through voltage-dependent calcium channels, which could increase the current inactivation rate of L-type voltage-dependent calcium channels to a certain extent, thereby reducing calcium influx; propofol could bind to GABAa receptor-specific sites, not only to increase the frequency of the opening of chloride channel by GABA, but also to enhance the binding of GABA binding sites with low affinity to GABA by positive allosteric regulation; propofol can inhibit the production of inflammatory cytokines such as TNF, IL-1 and IL-6 in the blood of patients with sepsis and had a strong inhibitory effect even at lower concentrations; propofol could inhibit the expression of the pro-apoptotic gene caspase-3 mRNA and enhance the expression of the anti-apoptotic gene Bcl-2 mRNA in brain tissues; propofol could competitively bind to membrane phospholipids, and form a stable phenoxy moiety with peroxide, which in fact forms free radicals of low activities in place of the free radicals of high activities, thereby reducing the lipid peroxidation cascade induced by the latter. The above results suggest that the mechanism by which propofol fight ischemic stroke may include anti-free radical effect, inhibition of lipid peroxidation, inhibition of intracellular calcium overload, and inhibition of cellular apoptosis. However, the clinical use of propofol in the treatment of ischemic stroke is restricted due to the general anesthetic effect thereof.

### Summary of the Invention

It is an object of the present invention to provide novel use of 3,3',5,5'-tetraisopropyl-4,4'-biphenol and a pharmaceutically acceptable salt, ester, or solvate thereof.

Therefore, the present invention provides the use of 3,3',5,5'-tetraisopropyl-4,4'-biphenol and a pharmaceutically acceptable salt, ester, or solvate thereof in the preparation of a medicament for the treatment and/or prevention of ischemic stroke. The structure of 3,3',5,5'-tetraisopropyl-4,4'-biphenol is as shown in formula (I):

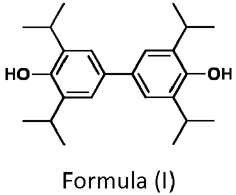

The 3,3',5,5'-tetraisopropyl-4,4'-biphenol according to the present invention further encompasses pharmaceutically acceptable salts, esters, or solvates of the compound.

The inventors have found under investigation that the biphenol had a strong affinity for GABA receptors and GABA agonizing effect, and was also able to antagonize NMDA receptors, regulate calcium channels, and limit calcium influx in cells, with more potent antioxidant and free radical scavenging effects than propofol. The Biphenol may act against ischemic stroke injury by various mechanisms. Most importantly, the biphenol does not cause loss of consciousness and is therefore of great value in clinical application for the treatment of various ischemic stroke symptoms.

The ester according to the present invention is a monoester or diester of 3,3',5,5'-tetraisopropyl-4,4'-biphenol. Preferably, the ester is a monoethyl ester represented by formula (II) or a diethyl ester represented by formula (III):

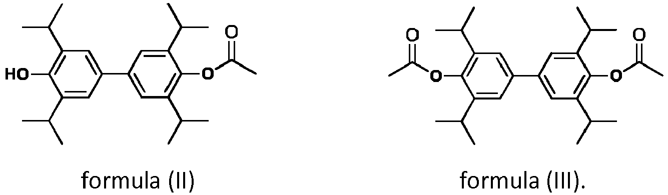

The "monoester or diester of 3,3',5,5'-tetraisopropyl-4,4'-biphenol" according to the present invention refers to a monoester or diester formed on the 4- and/or 4'-phenolic hydroxyl groups of said 3,3',5,5'-tetraisopropyl-4,4'-biphenol.

The term "treatment and/or prevention of ischemic stroke" as used in the present invention generally refers to the treatment and/or prevention of damage caused by ischemic stroke. The term "treatment and/or prevention" as used in the present invention means "therapeutic treatment and/or preventative treatment".

The pharmaceutically acceptable salt according to the present invention is a salt formed by 3,3',5,5'-tetraisopropyl-4,4'-biphenol with organic acids, inorganic acids or alkali metals; the pharmaceutically acceptable salts is, for example, sulfate, phosphate, hydrochloride, hydrobromide, acetate, oxalate, citrate, succinate, gluconate, tartrate, p-toluenesulfonate, benzenesulfonate, methanesulfonate, benzoate, lactate, maleate, lithium salt, sodium salt, potassium salt, or calcium salt.

According to particular embodiments of the present invention, in the use according to the present invention, 3,3',5,5'-tetraisopropyl-4,4'-biphenol and a pharmaceutically acceptable salt, ester, or solvate thereof can be formulated into a pharmaceutical composition, and the pharmaceutical composition comprises 3,3',5,5'-tetraisopropyl-4,4'-biphenol or a pharmaceutically acceptable salt, ester, or solvate thereof and a pharmaceutical excipient.

According to particular embodiments of the present invention, in the use according to the present invention, 3,3',5,5'-tetraisopropyl-4,4'-biphenol and a pharmaceutically acceptable salt, ester, or solvate thereof is formulated into a tablet, a capsule, injection, emulsion, liposome, lyophilized powder or microsphere formulation containing them; the capsule is, for example, a soft capsule.

According to particular embodiments of the present invention, in the use according to the present invention, the treatment and/or prevention of ischemic stroke is achieved by: improving cerebral ischemia and/or reperfusion neurological impairment; reducing cerebral ischemia and/or reperfusion cerebral infarction volume; reducing the endogenous oxygen free radical scavenger SOD consumption in brain tissues, reducing lipid peroxidation damage, while reducing serum MDA content; down-regulating cellular Fas expression in brain tissues; inhibiting brain cell apoptosis; and/or down-regulating cellular IL-1β and TNF-α expression in brain tissues.

According to particular embodiments of the present invention, in the use according to the present invention, the ischemic stroke includes damage caused by one or more of the following conditions: cerebral thrombosis, transient ischemic attack, basal ganglia infarction, atherosclerotic thrombotic cerebral infarction, lacunar cerebral infarction, cerebral embolism, and cerebrovascular dementia.

The present invention provides a method for treating and/or preventing ischemic stroke in animal or human comprising administering to an animal or human subject an effective amount of 3,3',5,5'-tetraisopropyl-4,4'-biphenol and a pharmaceutically acceptable salt, ester, or solvate thereof, and the structure of 3,3',5,5'-tetraisopropyl-4,4'-biphenol is as shown in formula (I):

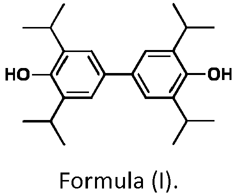

According to particular embodiments of the present invention, in the method of treating and/or preventing ischemic stroke in animal or human according to the present invention, the ester is a monoethyl ester or a diethyl ester of 3,3',5,5'-tetraisopropyl-4,4'-biphenol. Preferably, the ester is a monoethyl ester represented by formula (II) or a diethyl ester represented by formula (III):

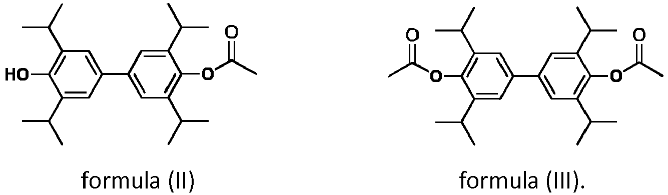

According to particular embodiments of the present invention, in the method of treating and/or preventing ischemic stroke in animal or human according to the present invention, the pharmaceutically acceptable salt is a salt formed by 3,3',5,5'-tetraisopropyl-4,4'-biphenol with organic acids, inorganic acids or alkali metals; the pharmaceutically acceptable salts is, for example, sulfate, phosphate, hydrochloride, hydrobromide, acetate, oxalate, citrate, succinate, gluconate, tartrate, p-toluenesulfonate, benzenesulfonate, methanesulfonate, benzoate, lactate, maleate, lithium salt, sodium salt, potassium salt, or calcium salt.

According to particular embodiments of the present invention, in the method of treating and/or preventing ischemic stroke in animal or human according to the present invention, 3,3',5,5'-tetraisopropyl-4,4'-biphenol and a pharmaceutically acceptable salt, ester, or solvate thereof can be formulated into a pharmaceutical composition, and the pharmaceutical composition comprises 3,3',5,5'-tetraisopropyl-4,4'- biphenol or a pharmaceutically acceptable salt, ester, or solvate thereof and a pharmaceutical excipient.

According to particular embodiments of the present invention, in the method of treating and/or preventing ischemic stroke in animal or human according to the present invention, 3,3',5,5'-tetraisopropyl-4,4'-biphenol and a pharmaceutically acceptable salt, ester, or solvate thereof is formulated into a tablet, a capsule, injection, emulsion, liposome, lyophilized powder or microsphere formulation containing them; the capsule is, for example, a soft capsule.

According to particular embodiments of the present invention, in the method of treating and/or preventing ischemic stroke in animal or human according to the present invention, the treatment and/or prevention of ischemic stroke is achieved by: improving cerebral ischemia and/or reperfusion neurological impairment; reducing cerebral ischemia and/or reperfusion cerebral infarction volume; reducing the endogenous oxygen free radical scavenger SOD consumption in brain tissues, reducing lipid peroxidation damage, while reducing serum MDA content; down-regulating cellular Fas expression in brain tissues; inhibiting brain cell apoptosis; and/or down-regulating cellular IL-1β and TNF-α expression in brain tissues.

According to particular embodiments of the present invention, in the method of treating and/or preventing ischemic stroke in animal or human according to the present invention, the ischemic stroke includes damage caused by one or more of the following conditions: cerebral thrombosis, transient ischemic attack, basal ganglia infarction, atherosclerotic thrombotic cerebral infarction, lacunar cerebral infarction, cerebral embolism, and cerebrovascular dementia.

The 3,3',5,5'-tetraisopropyl-4,4'-biphenol according to the present invention is commercially available or can be prepared by the method provided in the present invention.

Another object of the present invention is to provide a process for the preparation of 3,3',5,5'-tetraisopropyl-4,4'-biphenol.

Therefore, the present invention provides a process for the preparation of 3,3',5,5'-tetraisopropyl-4,4'-biphenol comprising the step of preparing 3,3',5,5'-tetraisopropyl-4,4'-biphenol from a compound represented by formula (IV)

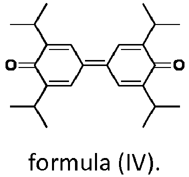

According to particular embodiments of the present invention, in the preparation process according to the present invention, 3,3',5,5'- tetraisopropyl-4,4'-biphenol is prepared by dissolving the compound represented by the formula (IV) in an organic solvent, preferably ethyl acetate, and then reacting it with an aqueous solution containing sodium hydrosulfite. Preferably, the aqueous solution containing sodium hydrosulfite is an aqueous solution formed of sodium hydrosulfite and sodium hydroxide.

According to particular embodiments of the present invention, in the preparation process according to the present invention, the compound represented by the formula (IV) is obtained by dissolving propofol in an organic solvent, preferably ethyl acetate, and then adding an inorganic salt thereto. Preferably, the inorganic salt is silver carbonate and anhydrous magnesium sulfate.

According to particular embodiments of the present invention, in the preparation process according to the present invention, the preparation of 3,3',5,5'-tetraisopropyl-4,4'-biphenol includes the following steps:
Propofol is dissolved in an organic solvent, preferably ethyl acetate, and even preferably with a mass/volume ratio of propofol to acetic acid of 1g: 4∼6 mL; inorganic salt is added thereto followed by stirring at room temperature; preferably, the inorganic salt includes silver carbonate and anhydrous magnesium sulfate; further preferably, the molar ratio of propofol to silver carbonate is 1.1-1.4:1, and the molar ratio of propofol to anhydrous magnesium sulfate is 1.2-1.4:1;
After the reaction is complete, water is added to the reaction solution, the solid is filtered and washed (preferably washed with ethyl acetate), the aqueous phase is removed, the ethyl acetate phase is dried (preferably dried over anhydrous sodium sulfate) and filtered, and the filtrate is evaporated to dryness (preferably evaporated under reduced pressure to dryness), optionally washed (preferably with anhydrous methanol), to give rosy red crystal;
The above rosy red solid is dissolved in ethyl acetate and mixed with the sodium hydrosulfite solution, preferably the sodium hydrosulfite solution being an aqueous solution of sodium hydrosulfite and NaOH, and optionally stirred; the ethyl acetate phase is then separated, and the aqueous phase is extracted several times (preferably the aqueous phase is extracted with ethyl acetate twice), dried (preferably dried over anhydrous sodium sulfate) and filtered, and the filtrate is evaporated to dryness (preferably evaporated under reduced pressure to dryness) to give a light yellow solid which is washed (preferably washed with petroleum ether) to afford 3,3',5,5'-tetraisopropyl-4,4'-biphenol as white solid.

According to particular embodiments of the present invention, in the preparation process according to the present invention, the process for the preparation of 3,3',5,5'-tetraisopropyl-4,4'-biphenol includes the following steps:
Propofol is dissolved in an organic solvent (preferably ethyl acetate), and an inorganic salt (preferably silver carbonate and anhydrous magnesium sulfate) is added thereto and stirred at room temperature;
After the reaction is complete, water is added to the reaction solution, the solid is filtered and washed (preferably with ethyl acetate), the aqueous phase is removed, the ethyl ester phase is dried (preferably over anhydrous sodium sulfate) and filtered, and the filtrate is evaporated (preferably under reduced pressure) to dryness, optionally washed (preferably adding anhydrous methanol), to give rosy red crystal;
The above rosy red solid is dissolved in ethyl acetate and mixed with the sodium hydrosulfite solution (preferably an aqueous solution of sodium hydrosulfite and NaOH), optionally stirred, the ethyl acetate phase is then separated; the aqueous phase is extracted (preferably with ethyl acetate) several times, preferably twice, dried (preferably over anhydrous sodium sulfate) and filtered, and the filtrate is evaporated (preferably under reduced pressure) to dryness to give a light yellow solid which is washed (preferably with petroleum ether) to provide a white solid.

Another object of the present invention is to provide a process for the preparation of 3,3',5,5'-tetraisopropyl-4,4'-biphenol ester.

In this regard, the present invention provides a process for the preparation of monoethyl ester of formula (II) which comprises the following steps:
4'-benzyloxy-3,3',5,5'-tetraisopropylbiphenyl-4-acetate is dissolved in an organic solvent, preferably methanol, at room temperature, and a catalyst, preferably palladium-carbon, is further added thereto, evacuated, and hydrogen is then charged therein; after sealing, a reaction is carried out at room temperature followed by filtering, and the filtrate is evaporated; the filtrate is preferably evaporated under reduced pressure to give a white solid, i.e., 4'-hydroxy-3,3',5,5'-tetraisopropylbiphenyl-4-acetate.

The present invention also provides a process for the preparation of diethyl esters of formula (III) which comprises the following steps:
4,4'-dihydroxy-3,3',5,5'-tetraisopropylbiphenyl is added to acetic anhydride, and a reaction is carried out at reflux under nitrogen; the reaction solution is cooled to room temperature, acetic anhydride is removed, preferably removed under reduced pressure, and water is added to the residue to provide a white solid which is then washed (preferably with ethanol and water) and dried to give 3,3',5,5'-tetraisopropylbiphenyl-4',4-diacetate.

The present invention also provides a pharmaceutical composition comprising 3,3',5,5'-tetraisopropyl-4,4'-biphenol and a pharmaceutically acceptable salt, ester, or solvate thereof, and the pharmaceutical composition comprises at least one of 3,3',5,5'-tetraisopropyl-4,4'-biphenol or a pharmaceutically acceptable salt, ester, or solvate thereof.

If desired, one or more pharmaceutically acceptable carriers or excipients may be further added into the pharmaceutical compositions according to the present invention.

In the pharmaceutical composition according to the present invention, 3,3',5,5'-tetraisopropyl-4,4'-biphenol or a pharmaceutically acceptable salt, ester, or solvate thereof may account for 0.1 to 99.9% by weight, with the balance being pharmaceutically acceptable carrier.

The pharmaceutical compositions according to the present invention may be formulated into any pharmaceutically acceptable dosage forms including tablets, sugar-coated tablets, film-coated tablets, enteric-coated tablets, capsules, hard capsules, soft capsules, oral liquid, oral agents, granules, electuary, pills, powders, paste, pellets, suspensions, powders, solution, injection, suppositories, ointment, plaster, cream, spray, drops, and patches. The formulations according the present invention are preferably tablets, capsules, injection, emulsion, liposomes, lyophilized powders or microsphere formulations. The capsules are, for example, soft capsules.

An orally administered formulation of the pharmaceutical composition according to the present invention may contain conventional excipients such as binders, fillers, diluents, tablets, lubricants, disintegrants, colorants, flavoring agents, and wetting agents. If necessary, tablets may be coated.

Suitable fillers include cellulose, mannitol, lactose and the like. Suitable disintegrants include starch, polyvinylpyrrolidone, and starch derivatives such as sodium starch glycolate. Suitable lubricants include, for example, magnesium stearate. Suitable pharmaceutically acceptable wetting agents include sodium dodecyl sulfate.

Solid oral compositions can be prepared by conventional methods such as mixing, filling, tabletting and the like. Repeated mixing allows the active agents to be distributed throughout the composition with a large amount of filler.

The form of the oral liquid formulation may be, for example, an aqueous or oily suspension, solution, emulsion, syrup, or elixir, or may be a dry product that can be reconstituted with water or other suitable carriers prior to use. Such liquid formulations may contain conventional additives such as suspending agents, emulsifying agents, non-aqueous carriers, preservatives and the like, and may contain conventional flavoring or coloring agents, if desired. The suspending agent is, for example, sorbitol, syrup, methylcellulose, gelatin, hydroxyethylcellulose, carboxymethylcellulose, aluminum stearate gel, and/or hydrogenated edible fats. The emulsifier is, for example, lecithin, sorbitan monooleate and/or arabic gum. The non-aqueous carriers (which may include edible oils) are, for example, almond oil, fractionated coconut oil, oily esters such as glycerol esters, propylene glycol and/or ethanol. The preservatives are, for example, parabens or propylparaben and/or sorbic acid.

As for injection, a liquid unit dosage form as prepared contains the active agent of the present invention (i.e., 3,3',5,5'-tetraisopropyl-4,4'-biphenol according to the present invention and a pharmaceutically acceptable salt, ester, or solvate thereof) and a sterile carrier. Depending on the carrier and concentration, the compound may be suspended or dissolved therein. The solution is usually prepared by dissolving the active agent in a carrier, which is filtered and sterilized before being loaded into a suitable vial or ampoule and then sealed. An adjuvant such as a local anesthetic, a preservative, and a buffer may also be dissolved in such a carrier. In order to improve its stability, the composition may be frozen after being loaded in a vial and water is removed under vacuum.

A suitable pharmaceutically acceptable carrier may be optionally added to the pharmaceutical composition according to the present invention when prepared as a medicament. The pharmaceutically acceptable carrier is selected from mannitol, sorbitol, sodium metabisulfite, sodium bisulfite, sodium thiosulfate, cysteine hydrochloride, mercaptoacetic acid, methionine, vitamin C, EDTA disodium, EDTA calcium sodium, monovalent alkali metal carbonate, acetate, phosphate or aqueous solution thereof, hydrochloric acid, acetic acid, sulfuric acid, phosphoric acid, amino acid, sodium chloride, potassium chloride, sodium lactate, xylitol, maltose, glucose, fructose, dextran, glycine, starch, sucrose, lactose, mannitol, silicon derivatives, cellulose and its derivatives, alginate, gelatin, polyvinylpyrrolidone, glycerol, Tween 80, agar, calcium carbonate, calcium bicarbonate, surfactant, polyethylene glycol, cyclodextrin, β-cyclodextrin, phospholipid materials, kaolin, talc, calcium stearate and/or magnesium stearate.

Preferably, the pharmaceutical excipients according to the present invention may include polyethylene glycol, phospholipids, vegetable oils, vitamin E and/or glycerol;
The phospholipid may be selected from one or more of soybean phospholipids, egg yolk lecithin and hydrogenated phospholipids;
The vegetable oil may be selected from one or more of soybean oil, olive oil and safflower oil.

The ischemic stroke described in the present invention relates to the following conditions: cerebral thrombosis, transient ischemic attack, basal ganglia infarction, atherosclerotic thrombotic cerebral infarction, lacunar infarction, cerebral embolism, and brain vascular dementia. The above conditions usually cause headache, dizziness, tinnitus, hemiplegia, swallowing difficulty, babbling, nausea, vomiting, coma and the like.

The use of the present invention is realized by improving the neurological impairment upon ischemic reperfusion.

The use of the present invention is realized by reducing the volume of ischemic reperfusion cerebral infarction.

The use of the present invention is realized by reducing the consumption of endogenous oxygen free radical scavenger SOD, reducing lipid peroxidation damage, and at the same time lowering the serum MDA content.

The use of the present invention is realized by effectively down-regulating the cellular expression of Fas in brain tissues.

The use of the present invention is realized by effectively inhibiting brain cell apoptosis.

The use of the present invention is realized by effectively down-regulating the cellular expression of IL-1β and TNF-α in brain tissues.

In the Examples of the present invention, a middle cerebral artery occlusion animal model (MCAO) established by the inventor using the suture method has the advantages of no craniotomy, less trauma, accurate control of the ischemia and reperfusion time, and is currently the most classic model of focal cerebral ischemic reperfusion. This model has been widely used domestically and abroad in cerebral ischemia experiments and evaluation of medicaments for treating cerebral ischemic reperfusion injury.

Upon cerebral ischemic reperfusion, intracellular oxygen free radicals increase significantly and are particularly prone to attack biomembrane structures comprising unsaturated double bonds, so as to induce lipid peroxidation which disrupt the membrane structures, affect membrane permeability, and lead to a series of pathophysiological changes to ion transportation, bioenergy generation and organelle functions, resulting in damages to nerve cells, glial cells, and vascular endothelial cells. SOD is the primary enzymatic defense mechanism against intracellular oxygen free radicals, which scavenges the superoxide anion radicals by disproportionation. Change in the content of MDA, a metabolite of the lipid peroxidation reaction of oxygen free radicals with biomembrane unsaturated fatty acids, indirectly reflects the content of oxygen free radicals and the degree of cell damage in tissues. Therefore, determination of the SOD activity and the MDA content in ischemic reperfusion can reflect the extent of the lipid peroxidation reaction induced by free radicals in vivo.

Cerebral ischemic reperfusion injury is primarily related to response to oxidative stress, inflammatory response, calcium overload, cerebral edema, and apoptosis. Upon cerebral ischemic reperfusion, due to energy metabolism and the action of various endogenous active substances, Ca²⁺ release from the reservoir is stimulated and intracellular Ca²⁺ concentration increase. Also, cerebral ischemia can cause EAA to be excessively released from neuronal or glial cell transmitter pool or metabolic pool. EAA can induce intracellular Ca²⁺ overload, resulting in increased free radical generation. The increase of free radicals and EAA may both induce the expression of apoptotic factors such as Fas after cerebral ischemic reperfusion, thereby promoting cell apoptosis. As such, cell apoptosis status can also reflect the degree of damage to brain cells. IL-1β and TNF-α are the major proinflammatory factors after brain injury and participate in the inflammatory response in ischemic and reperfusion regions. After cerebral ischemic reperfusion, the inflammatory cells in the vicinity of the endothelial cells, neurons, astrocytes, and blood vessels in the injured area are activated, triggering an inflammatory response by releasing IL-1β and TNF-α and therefore resulting in neuronal damage. Measurement of the contents of IL-1β and TNF-α as the starting factors in inflammatory response is of great significance for the evaluation of brain injury after ischemic reperfusion.

Meanwhile, because the occurrence of reperfusion in stroke patients is often delayed, the brain tissue ischemic "hunger" injury cannot be ignored either. Thus, the present invention also evaluate the protective effect of biphenol in permanent ischemic injury by using the middle cerebral artery occlusion animal model (MCAO) established by the suture method.

It is demonstrated with experiments in the present invention: 3,3',5,5'-tetraisopropyl-4,4'-biphenol and a pharmaceutically acceptable salt, ester, or solvate therefor can effectively reduce the consumption of the endogenous oxygen free radical scavenger SOD activity due to cerebral ischemic reperfusion injury, reduce lipid peroxidation damage, reduce serum MDA content, effectively down-regulate Fas expression, reduce apoptotic cells, and reduce the expression of the proinflammatory cytokines IL-1β and TNF-α, so as to achieve protection of the rat neurons against cerebral ischemic reperfusion, and also have a protective effect on permanent cerebral ischemic injury.

### Detailed Description of the Invention

The present invention will be further described with reference to the following examples. It is to be understood that these examples are merely illustrative and are not intended to limit the scope of the invention.

### Example 1. Compound 1: 3,3',5,5'-tetraisopropyl-4,4'-biphenol

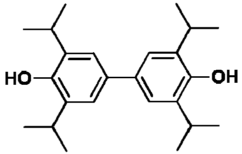

20g propofol was weighed and dissolved in 100mL ethyl acetate, 24.75g silver carbonate and 10g anhydrous magnesium sulfate were then added thereto, stirred at room temperature for 2h, and the reaction was checked for completion. Water was added to the reaction solution until no bubble emerged. The solid was filtered and washed with ethyl acetate, and the aqueous phase was removed. The ethyl acetate phase was dried over anhydrous sodium sulfate for 1h and filtered. The filtrate was evaporated to dryness under reduced pressure and washed with anhydrous methanol to give 12.30g rosy red crystal. 7g of the above rosy red solid was dissolved in 100mL of ethyl acetate. Then, 27.66g sodium hydrosulfite was dissolved in 1 mol/L NaOH and added to the resultant ethyl acetate solution of the above rosy red solid, the mixture was stirred at room temperature for 1.5 h, and the reaction was checked for completion. The ethyl acetate phase was separated, the aqueous phase was extracted twice with ethyl acetate, dried over anhydrous sodium sulfate and filtered, and the filtrate was evaporated to dryness under reduced pressure to give 5g of a light yellow solid which was washed with petroleum ether to give 4.5g white solid. ¹H NMR (300 MHz, CDCl₃): δ 7.22 (s, 4H), 4.81 (s, 2H), 3.27-3.20 (m, 4H), 1.37-1.35(d, 24H).

### Example 2. Compound 2: 4'-hydroxy-3,3',5,5'-tetraisopropylbiphenyl- 4-acetate

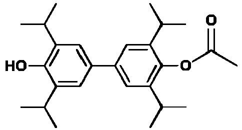

4'-Benzyloxy-3,3',5,5'-tetraisopropylbiphenyl-4-acetate (5g, 10.27 mmol) was dissolved in 200 mL methanol at room temperature, 10% palladium-carbon (570 mg) was added thereto, followed by evacuation to vacuum and charging with hydrogen, which was repeated for three times, and then sealed and reacted at room temperature for 10h. The palladium-carbon in the reaction solution was filtered, and the filtrate was evaporated under reduced pressure to obtain 4'-hydroxy-3,3',5,5'-tetraisopropylbiphenyl-4-acetate (3.9 g, 95.73%) as a white solid.¹H NMR (300 MHz, CDCl₃) δ 7.19 (s, 4H), 4.86 (s, 1H), 3.37-3.32 (m, 4H), 3.16 (s, 3H), 1.20 (d, 24H).

### Example 3. Compound 3: 3,3',5,5'-tetraisopropylbiphenyl-4'-diacetate

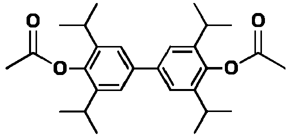

4,4'-dihydroxy-3,3',5,5'-tetraisopropylbiphenyl (5 g, 14.10 mmol) was added to 30 mL acetic anhydride and allowed to reflux for 3h under nitrogen. The reaction solution was cooled to room temperature, and the acetic anhydride was removed under reduced pressure. Water (200 mL) was added to the residue to give a white solid which was washed with 10% cold ethanol (100 mL) and water (200 mL) and dried to afford 3,3',5,5'-tetraisopropylbiphenyl-4'-diacetate (6 g, 95.06%) as a white solid.
White solid, ¹H NMR (300 MHz, CDCl₃) δ 7.19 (s, 4H), 2.91-2.89 (m, 4H), 2.32 (s, 6H), 1.19 (d, 24H).

### Example 4. Effects of compound 1-3 on neurological impairment score, cerebral infarct volume, Fas, IL-1β, and TNF-α in brain tissues, and cell apoptosis in rats with cerebral ischemic reperfusion injury

### (1) Material:

An aqueous solution of compound 1-3 in PEG400 at a concentration of 10 mg/mL, in which the concentration of PEG400 in the PEG400 aqueous solution was 400 mg/ml; GL-22M low temperature centrifuge (Hubei Saite Xiangyi); BI2000 image analyzer (Chengdu Techman Software Co.,Ltd.), SOD and MDA assay kits (Nanjing Jiancheng Bioengineering Research Institute); Fas and TUNEL kits (Wuhan Boside Biological Engineering Co., Ltd.), IL-1β and TNF-α kits (Shanghai HengYuan Biotechnology Co., Ltd.); other reagents were made in China, analytically pure.

### (2) Method:

### 1. Experimental animals and grouping

120 healthy male SD rats weighing 250-300g, provided by the Experimental Animal Center of the Fourth Military Medical University, were randomly divided into 5 groups: the sham operation group, the ischemic reperfusion group, the Example 1 group (using compound 1 as the test sample), the Example 2 group (using compound 2 as the test sample) and the Example 3 group (compound 3 as the test sample).

### 2. Animal model preparation and treatment

Rats were intraperitoneally injected with 10% chloral hydrate 350mg/kg after anesthesia and opened up at the center of the neck in accordance with the Zea Longa modified method. The right common carotid artery was separated, and the right external carotid artery branches was ligated. A small opening was made at the distal end of the external carotid artery, and a previously prepared thread was inserted through the common carotid artery and the external carotid artery bifurcation into the internal carotid artery, till the anterior end of the middle cerebral artery, with an immersed depth of 18 to 19mm. The thread was then secured, and the wound was sutured layer by layer. After the operation, the rats were placed in a clean incubator to wake up. The criteria for a successfully prepared cerebral ischemia model was: after waking up, the rats showed Horner syndrome on the right side and hemiplegia on the left side. The sham operation group, the ischemic reperfusion group, and the Example groups were prepared strictly according to the requirements of the cerebral ischemic reperfusion model, while in the sham operation group the thread was put only into the external carotid artery. The animals were allowed to eat and drink freely after waking up. After 2h, the thread was pulled out to achieve reperfusion. The sham operation group, the ischemic reperfusion group, and the Example groups were administered intravenously the aqueous solution of compound 1-3 in PEG400 at 40mg/kg (40 mg/kg means that 40 mg of the test compound per kg of the body weight of rats) 30min before reperfusion and 12h after reperfusion, respectively. The sham operation group and the ischemic reperfusion group were injected with the same amount of blank PEG400 aqueous solution at the same time point.

### 3. Neurological impairment score in rats

The neurological impairment was scored in each group 24 h after reperfusion. The scoring was in accordance with the Longa 5-grade method: grade 0: no nerve injury symptom; grade 1: inability of stretching the contralateral forepaw; grade 2: circling to the opposite side; grade 3: tumbling to the opposite side; grade 4: no autonomous activity with loss of consciousness.

### 4. Sample collection and preparation

24 hours after reperfusion, 8 rats in each group were sacrificed by decapitation, the brain was removed, rinsed with PBS (pH 7.4) at -20 °C for 20min, evenly sliced (with a thickness of 2mm), stained in a 2% TTC solution at 37°C in the dark for 30min, fixed in 10% formalin for 24h, and photographs were taken to analyze the infarct volume. A mixture of femoral arterial and venous blood was drawn from another 8 rats in each group under anesthesia, placed in a low temperature centrifuge at 4 °C, centrifuged at 3500r/min for 20min, and the supernatant was stored in a refrigerator at -20 °C ready for SOD and MDA detection. After the blood was drawn, a perfusion needle was insert through the apex to the ascending aorta, and saline at 4 °C was rapid infused until the effluent became clear, followed by 4% paraformaldehyde phosphate buffer perfusion for fixation; the brain was removed by craniotomy, and brain tissues 2mm in front of and behind the optic chiasma were taken and fixed, dehydrated, rendered transparent, impregnated in wax, and embedded. Brain continuous coronary tissue pathological sections were consecutively cut for later use. The remaining 8 rats in each group were sacrificed, brains were removed, the ischemic brain hemispheres were immediately taken on an ice tray, and 10% brain tissue homogenate was prepared for the detection of IL-1β and TNF-α.

### 5. Determination of relative infarct volume

The infarct area in each brain section was analyzed and quantified by the imageJ image software, and the ratio of cerebral infarction volume in the overall brain volume was calculated.

### 6. SOD and MDA determination

SOD and MDA were determined strictly in accordance with instructions of the kit.

### 7. Fas determination

The immunohistochemical method was used for determination. Brain tissue paraffin sections were dewaxed into water, washed with 3% H₂O₂ to eliminate endogenous peroxidase activity, and rinsed with distilled water for 3 times. Sodium citrate buffer was used for antigen heat repairing, and blocking with calf serum was carried out at room temperature for 15min; a rabbit anti-mouse Fas antibody was added dropwise, allowed to stand at 4°C overnight, biotinylated goat anti-rabbit IgG was added dropwise, and heated in a water bath at 37°C for 20min. Washing with PBS for 5min was continuously repeated 4 times, followed by staining with DAB and sufficient washing without counterstaining. The sections were then gradient dehydrated with alcohol, made transparent with toluene, sealed and fixed. Images were captured by a video camera under high-power optical microscope and inputted into the image analysis system for image analysis. Five non-overlapping fields were randomly selected from each section. Five areas in each field were selected for determination of gray scale. The average gray scale was calculated, and the average gray scale was inversely proportional to the rate of positive expression.

### 8. Apoptosis determination

The TUNEL method was used for determination. Brain tissue paraffin sections were dewaxed into water, washed with 3% H₂O₂ to eliminate endogenous peroxidase activity, and rinsed with distilled water for 2 min, repeated 3 times. A labelling solution was added for labelling at 37°C for 2h, and a blocking solution was added for blocking at room temperature for 30min. A biotinylated anti-digoxin antibody was added for a reaction at 37°C for 30min, and SABC was added for a reaction at 37°C for 30min. The sections were continuously rinsed with TBS for 5minrepeatedly for 4 times, stained with DAB, sufficiently washed, mild counterstained with Hematoxylin, gradient dehydrated with alcohol, made transparent with toluene, sealed and fixed. Five non-overlapping fields in semi-dark band were randomly selected from each section and inputted into the image analysis system. The number of apoptotic cells was counted, and the average was designated the number of apoptotic cells.

### 9. Determination of IL-1β and TNF-α

Brain tissue homogenate was hypothermally centrifuged at 3000rpm for 15min, the supernatant was taken, and IL-1β and TNF-α were determined in strict accordance with instructions of the kits.

### (3) Results:

Compounds 1-3 can substantially improve the neurological impairment in rats with ischemic reperfusion, significantly reduce the cerebral infarction volume in rats with ischemic reperfusion, significantly reduce the consumption of the endogenous oxygen free radical scavenger SOD, reduce lipid peroxidation damage while reducing the serum MDA content, effectively down-regulate cellular Fas expression in brain tissues, effective inhibit brain cell apoptosis, and also effectively down-regulate cellular IL-1β and TNF-α expression in brain tissues in rats. The results are shown in Table 1 below.

**Table 1**

| | Sham operation group | Ischemic reperfusion group | Example 1 group | Example 2 group | Example 3 group |
|---|---|---|---|---|---|
| Average score of neurological impairment | 0 | 3.2 | 1.8 | 2.0 | 2.1 |
| Relative cerebral infarction volume (%) | 0 | 35.28±5.22 | 16.21±3.28* | 18.71±4.69* | 23.22±3.12* |
| SOD activity (U/mL) | 101.54±3.45 | 82.14±4.37 | 97.16±3.85* | 96.78±2.94* | 94.46±2.19* |
| MDA content (mmol/mL) | 2.49±0.66 | 7.22±0.61 | 4.35±0.52* | 4.74±0.67* | 5.44±0.49* |
| Fas average gray scale | 175.96±5.14 | 134.33±6.18 | 165.11±5.02 * | 162.47±3.96 * | 148.36±5.33 * |
| Number of apoptotic cells | 4.62±1.54 | 37.26±4.10 | 17.32±4.49* | 19.14±4.24* | 24.65±3.41* |
| IL-1β content (ng/mL) | 0.39±0.08 | 0.92±0.14 | 0.60±0.08* | 0.63±0.12* | 0.79±0.11* |
| TNF-α content (ng/mL) | 2.74±0.21 | 6.47±0.65 | 4.01±0.82* | 4.11±0.72* | 5.03±0.59* |

In comparison to the ischemic reperfusion group *: p<0.05

### Example 5. Effects of compound 1-3 on neurological impairment score, cerebral infarct volume, Fas, IL-1β, and TNF-α in brain tissues, and cell apoptosis in rats with permanent cerebral ischemic reperfusion injury

### (1) Material:

An aqueous solution of compound 1-3 in PEG400 at a concentration of 10 mg/mL, in which the concentration of PEG400 in the PEG400 aqueous solution was 400 mg/ml; GL-22M low temperature centrifuge (Hubei Saite Xiangyi); BI2000 image analyzer (Chengdu Techman Software Co.,Ltd.), SOD and MDA assay kits (Nanjing Jiancheng Bioengineering Research Institute); Fas and TUNEL kits (Wuhan Boside Biological Engineering Co., Ltd.), IL-1β and TNF-α kits (Shanghai HengYuan Biotechnology Co., Ltd.); other reagents were made in China, analytically pure.

### (2) Method:

### 1. Experimental animals and grouping

120 healthy male SD rats weighing 250-300g, provided by the Experimental Animal Center of the Fourth Military Medical University, were randomly divided into 5 groups: the sham operation group, the permanent cerebral ischemia model group, the Example 1 group (using compound 1 as the test sample), the Example 2 group (using compound 2 as the test sample) and the Example 3 group (compound 3 as the test sample).

### 2. Animal model preparation and treatment

Rats were intraperitoneally injected with 10% chloral hydrate 350mg/kg after anesthesia and opened up at the center of the neck in accordance with the Zea Longa modified method. The right common carotid artery was separated, and the right external carotid artery branches was ligated. A small opening was made at the distal end of the external carotid artery, and a previously prepared thread was inserted through the common carotid artery and the external carotid artery bifurcation into the internal carotid artery, till the anterior end of the middle cerebral artery, with an immersed depth of 18 to 19mm. The thread was then secured, and the wound was sutured layer by layer. After the operation, the rats were placed in a clean incubator to wake up. The criteria for a successfully prepared cerebral ischemia model was: after waking up, the rats showed Horner syndrome on the right side and hemiplegia on the left side. The sham operation group, the permanent cerebral ischemia model group, and the Example groups were prepared strictly according to the requirements of the cerebral ischemic reperfusion model, while in the sham operation group the thread was put only into the external carotid artery. The animals were allowed to eat and drink freely after waking up. The sham operation group, the permanent cerebral ischemia model group, and the Example groups were administered intravenously the aqueous solution of compound 1-3 in PEG400 at 40mg/kg (40 mg/kg means that 40 mg of the test compound per kg of the body weight of rats) 30min before insertion of thread and 12h after embolism, respectively. The sham operation group and the permanent cerebral ischemia model group were injected with the same amount of blank PEG400 aqueous solution at the same time point.

### 3. Neurological impairment score in rats

The neurological impairment was scored in each group 24 h after the embolism. The scoring was in accordance with the Longa 5-grade method: grade 0: no nerve injury symptom; grade 1: inability of stretching the contralateral forepaw; grade 2: circling to the opposite side; grade 3: tumbling to the opposite side; grade 4: no autonomous activity with loss of consciousness.

### 4. Sample collection and preparation

24 hours after the embolism, 8 rats in each group were sacrificed by decapitation, the brain was removed, rinsed with PBS (pH 7.4) at -20 °C for 20min, evenly sliced (with a thickness of 2mm), stained in a 2% TTC solution at 37°C in the dark for 30min, fixed in 10% formalin for 24h, and photographs were taken to analyze the infarct volume. A mixture of femoral arterial and venous blood was drawn from another 8 rats in each group under anesthesia, placed in a low temperature centrifuge at 4 °C, centrifuged at 3500r/min for 20min, and the supernatant was stored in a refrigerator at -20 °C ready for SOD and MDA detection. After the blood was drawn, a perfusion needle was insert through the apex to the ascending aorta, and saline at 4 °C was rapid infused until the effluent became clear, followed by 4% paraformaldehyde phosphate buffer perfusion for fixation; the brain was removed by craniotomy, and brain tissues 2mm in front of and behind the optic chiasma were taken and fixed, dehydrated, rendered transparent, impregnated in wax, and embedded. Brain continuous coronary tissue pathological sections were consecutively cut for later use. The remaining 8 rats in each group were sacrificed, brains were removed, the ischemic brain hemispheres were immediately taken on an ice tray, and 10% brain tissue homogenate was prepared for the detection of IL-1β and TNF-α.

### 5. Determination of relative infarct volume

The infarct area in each brain section was analyzed and quantified by the imageJ image software, and the ratio of cerebral infarction volume in the overall brain volume was calculated.

### 6. SOD and MDA determination

SOD and MDA were determined strictly in accordance with instructions of the kit.

### 7. Fas determination

The immunohistochemical method was used for determination. Brain tissue paraffin sections were dewaxed into water, washed with 3% H₂O₂ to eliminate endogenous peroxidase activity, and rinsed with distilled water for 3 times. Sodium citrate buffer was used for antigen heat repairing, and blocking with calf serum was carried out at room temperature for 15min; a rabbit anti-mouse Fas antibody was added dropwise, allowed to stand at 4°C overnight, biotinylated goat anti-rabbit IgG was added dropwise, and heated in a water bath at 37°C for 20min. Washing with PBS for 5min was continuously repeated 4 times, followed by staining with DAB and sufficient washing without counterstaining. The sections were then gradient dehydrated with alcohol, made transparent with toluene, sealed and fixed. Images were captured by a video camera under high-power optical microscope and inputted into the image analysis system for image analysis. Five non-overlapping fields were randomly selected from each section. Five areas in each field were selected for determination of gray scale. The average gray scale was calculated, and the average gray scale was inversely proportional to the rate of positive expression.

### 8. Apoptosis determination

The TUNEL method was used for determination. Brain tissue paraffin sections were dewaxed into water, washed with 3% H₂O₂ to eliminate endogenous peroxidase activity, and rinsed with distilled water for 2 min, repeated 3 times. A labelling solution was added for labelling at 37°C for 2h, and a blocking solution was added for blocking at room temperature for 30min. A biotinylated anti-digoxin antibody was added for a reaction at 37°C for 30min, and SABC was added for a reaction at 37°C for 30min. The sections were continuously rinsed with TBS for 5minrepeatedly for 4 times, stained with DAB, sufficiently washed, mild counterstained with Hematoxylin, gradient dehydrated with alcohol, made transparent with toluene, sealed and fixed. Five non-overlapping fields in semi-dark band were randomly selected from each section and inputted into the image analysis system. The number of apoptotic cells was counted, and the average was designated the number of apoptotic cells.

### 9. Determination of IL-1β and TNF-α

Brain tissue homogenate was hypothermally centrifuged at 3000rpm for 15min, the supernatant was taken, and IL-1β and TNF-α were determined in strict accordance with instructions of the kits.

### (3) Results:

Compounds 1-3 can substantially improve the neurological impairment in permanent cerebral ischemia model rats, significantly reduce the cerebral infarction volume in permanent cerebral ischemia model rats, significantly reduce the consumption of the endogenous oxygen free radical scavenger SOD, reduce lipid peroxidation damage while reducing the serum MDA content, effectively down-regulate cellular Fas expression in brain tissues in permanent cerebral ischemia model rats, effective inhibit brain cell apoptosis, and also effectively down-regulate cellular IL-1β and TNF-α expression in brain tissues in permanent cerebral ischemia model rats. The results are shown in Table 2 below.

**Table 2**

| | Sham operation group | Permanent cerebral ischemia model group | Example 1 group | Example 2 group | Example 3 group |
|---|---|---|---|---|---|
| Average score of neurological impairment | 0 | 3.5 | 2.1 | 2.4 | 2.5 |
| Relative cerebral infarction volume(%) | 0 | 39.82±5.19 | 21.27±4.26* | 24.71±3.76* | 27.92±4.51* |
| SOD activity (U/mL) | 102.29±4.72 | 84.42±3.88 | 98.44±3.12* | 97.36±4.51* | 93.28±3.49* |
| MDA content (mmol/mL) | 2.45±0.67 | 7.05±0.57 | 4.16±0.52* | 4.49±0.59* | 5.10±0.82* |
| Fas average gray scale | 178.69± 4.53 | 126.7±7.82 | 161.31±5.27* | 158.28±6.66* | 149.63±8.15* |
| Number of apoptotic cells | 4.47±1.09 | 39.41±4.34 | 19.31±3.36* | 20.41±5.02* | 23.27±3.78* |
| IL-1β content (ng/mL) | 0.38±0.05 | 0.95±0.17 | 0.61±0.15* | 0.64±0.09* | 0.78±0.12* |
| TNF-α content (ng/mL) | 2.58±0.23 | 6.74±0.47 | 4.17±0.62* | 4.25±0.66* | 5.15±0.88* |

In comparison to the permanent cerebral ischemia model group *: p<0.05

### Example 6. Comparison of efficacy of the present invention over existing positive drugs

The therapeutic effects of propofol and edaravone on the ischemic reperfusion model and the permanent cerebral ischemia model rats were evaluated according to the methods described above in Examples 4 and 5 (propofol 15mg/kg; edaravone 3mg/kg; herein, 15 mg/kg means that the rats were given 15 mg of propofol per kilogram of body weight, and 3 mg/kg means the rats were given 3 mg of edaravone per kilogram of body weight). Also, the change of behavior of the rats were observed after administration. The experimental results were shown in the following Table 3 and Table 4, respectively.

**Table 3. Efficacy of positive drugs in ischemic reperfusion rat model**

| | Model group | Example 1 group | Propofol injection (100mg/10mL) | Edaravone injection (30mg/20mL) |
|---|---|---|---|---|
| Neurological impairment score | 3.2 | 1.8 | 2.5 | 2.6 |
| Relative cerebral infarct volume (%) | 35.28±5.22 | 16.21±3.28 | 27.14±5.08* | 29.31±3.95* |
| Fas average gray scale | 134.33±6.18 | 165.11±5.02 | 142.15±5.71* | 139.65±2.05* |
| SOD activity (U/mL) | 82.14±4.37 | 97.16±3.85 | 88.25±2.19* | 88.16±3.99* |
| MDA content (mmol/mL) | 7.22±0.61 | 4.35±0.52 | 5.58±0.48* | 6.17±0.54* |
| Number of apoptotic cells | 37.26±4.10 | 17.32±4.49 | 26.08±3.72* | 28.6±4.90* |
| IL-1β content (ng/mL) | 0.92±0.14 | 0.60±0.08 | 0.73±0.09* | 0.75±0.05* |
| TNF-α content (ng/mL) | 6.47±0.65 | 4.01±0.82 | 5.02±0.31* | 5.14±0.44* |
| Change of behavior of rats within 30 min after the administration 12h after reperfusion | Sober, no significant difference as compared to pre-administration | Sober, no significant difference as compared to pre-administration | Anesthetized, loss of righting reflex | Sober, no significant difference as compared to pre-administration |

| | | | | |
|---|---|---|---|---|
| *: Relative to the Example 1 group, p<0.05. | | | | |

The results in Table 3 showed that the therapeutic effect of the biphenyl derivatives on the model group was superior to that of the positive control drugs propofol and edaravone. Although most of the Examples show significant advantages over the efficacy of the positive drugs (p<0.05), Table 3 lists only the efficacy experimental results of Example 1 in comparison to the positive drugs as a reference. Further, it was found that the rats lost righting reflex after propofol administration and entered an anesthetic state while the rats in the other administration groups did not show obvious change of behavior.

**Table 4. Efficacy of positive drugs in permanent cerebral ischemia rat model**

| | Model group | Example 1 group | Propofol injection (100mg/10mL) | Edaravone injection (30mg/20mL) |
|---|---|---|---|---|
| Neurological impairment score | 3.5 | 2.1 | 2.7 | 2.8 |
| Relative cerebral infarct volume (%) | 39.82±5.19 | 21.27±4.26 | 30.11±3.58* | 31.29±4.02* |
| Fas average gray scale | 126.7±7.82 | 161.31±5.27 | 140.51±4.84* | 138.95±5.50* |
| SOD activity (U/mL) | 84.42±3.88 | 98.44±3.12 | 89.13±3.82* | 90.62±3.81* |
| MDA content (mmol/mL) | 7.05±0.57 | 4.16±0.52 | 6.10±0.29* | 5.98±0.44* |
| Number of apoptotic cells | 39.41±4.34 | 19.31±3.36 | 31.06±3.55* | 33.19±3.81* |
| IL-1β content (ng/mL) | 0.95±0.17 | 0.61±0.15 | 0.78±0.07* | 0.76±0.04* |
| TNF-α content (ng/mL) | 6.74±0.47 | 4.17±0.62 | 5.40±0.32* | 5.53±0.54* |
| Change of behavior of rats within 30 min after the administration 12h after embolism | Sober, no significant difference as compared to pre-administration | Sober, no significant difference as compared to pre-administration | Anesthetized, loss of righting reflex | Sober, no significant difference as compared to pre-administration |

| | | | | |
|---|---|---|---|---|
| *: Relative to the Example 1 group, p<0.05. | | | | |

The results in Table 4 showed that the therapeutic effect of the biphenyl derivatives on the model group was superior to that of the positive control drugs propofol and edaravone. Although most of the Example groups show significant advantages over the efficacy of the positive drugs (p<0.05), Table 4 lists only the efficacy experimental results of Example 1 in comparison to the positive drugs as a reference. Further, it was found that the rats lost righting reflex after propofol administration and entered an anesthetic state while the rats in the other administration groups did not show obvious change of behavior.

### Example 7. Oil-based preparation

The formulation of the oil-based preparation of the biphenyl derivative of the present invention can be as shown in Table 5:

**Table 5**

| Components | Amount in formulation |
|---|---|
| 3,3',5,5'-tetraisopropyl-4,4'-biphenol | 200 mg |
| Tetrahydrofuran polyglycol ether | 0.80 ml |
| Vitamin E acetate | 5 mg |
| Benzyl alcohol | 50 µl |
| Castor oil | Add to 1 ml |

### Example 8. Tablet

The formulation of the tablet of the biphenyl derivative of the present invention can be as shown in Table 6:

**Table 6**

| Components | Amount in formulation |
|---|---|
| 3,3',5,5'-tetraisopropyl-4,4'-biphenyl diacetate | 200 mg |
| Lactose | 140 mg |
| Microcrystalline cellulose | 100 mg |
| Starch pulp | 50mg |
| Sodium carboxymethyl starch | 10mg |
| Magnesium stearate | 1mg |

### Example 9. Capsule

The formulation of the capsule of the biphenyl derivative of the present invention can be as shown in Table 7:

**Table 7**

| Components | Amount in formulation |
|---|---|
| 3,3',5,5'-tetraisopropyl-4,4'-biphenol | 1g |
| Olive oil | 10g |
| Egg yolk lecithin | 1.2g |
| Vitamin E | 0.2g |

### Example 10. Emulsion

The formulation of the emulsion of the biphenyl derivative of the present invention can be as shown in Table 8:

**Table 8**

| Components | Amount in formulation |
|---|---|
| 4'-hydroxy-3,3',5,5'-tetraisopropylbiphenyl-4-acetate | 1g |
| Soybean oil | 10g |
| Egg yolk lecithin | 1.2g |
| Vitamin E | 0.1g |
| Glycerin | 2.25g |
| Sodium hydroxide | Appropriate amount |
| Water for injection | Add to 100ml |

## Claims

1. Use of 3,3',5,5'-tetraisopropyl-4,4'-biphenol and a pharmaceutically acceptable salt, ester, or solvate thereof in the preparation of a medicament for the treatment and/or prevention of ischemic stroke, wherein the structure of 3,3',5,5'-tetraisopropyl-4,4'-biphenol is as shown in formula (I):

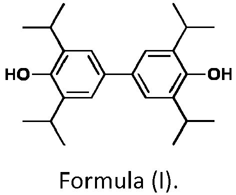

2. The use according to claim 1, wherein the ester is a monoester or diester of 3,3',5,5'-tetraisopropyl-4,4'-biphenol, and preferably the ester is a monoethyl ester represented by formula (II) or a diethyl ester represented by formula (III):

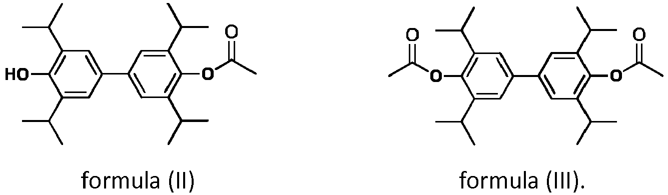

3. The use according to claim 1 or 2, wherein the pharmaceutically acceptable salt is a salt formed by 3,3',5,5'-tetraisopropyl-4,4'-biphenol with organic acid, inorganic acid, or alkali metal, and wherein the pharmaceutically acceptable salt is, for example, sulfate, phosphate, hydrochloride, hydrobromide, acetate, oxalate, citrate, succinate, gluconate, tartrate, p-toluenesulfonate, benzenesulfonate, methanesulfonate, benzoate, lactate, maleate, lithium salt, sodium salt, potassium salt, or calcium salt.

4. The use according to claim 1 or 2, wherein, 3,3',5,5'-tetraisopropyl-4,4'-biphenol and a pharmaceutically acceptable salt, ester, or solvate thereof can be formulated into a pharmaceutical composition which comprises the 3,3',5,5'-tetraisopropyl-4,4'-biphenol or a pharmaceutically acceptable salt, ester, or solvate thereof and a pharmaceutical excipient.

5. The use according to claim 1 or 2, wherein, 3,3',5,5'-tetraisopropyl-4,4'-biphenol and a pharmaceutically acceptable salt, ester, or solvate thereof is formulated into a tablet, a capsule, injection, emulsion, liposome, lyophilized powder or microsphere formulation containing it, and wherein the capsule is, for example, a soft capsule.

6. The use according to claim 1 or 2, wherein the treatment and/or prevention of ischemic stroke is achieved by improving cerebral ischemia and/or reperfusion neurological impairment; reducing cerebral ischemia and/or reperfusion cerebral infarction volume; reducing the endogenous oxygen free radical scavenger SOD consumption in brain tissues, reducing lipid peroxidation damage, while reducing serum MDA content; down-regulating cellular Fas expression in brain tissues; inhibiting brain cell apoptosis; and/or down-regulating cellular IL-1β and TNF-α expression in brain tissues.

7. The use according to claim 1 or 2, wherein the ischemic stroke includes damage caused by one or more of the following conditions: cerebral thrombosis, transient ischemic attack, basal ganglia infarction, atherosclerotic thrombotic cerebral infarction, lacunar cerebral infarction, cerebral embolism, and cerebrovascular dementia.

8. A method for treating and/or preventing ischemic stroke in animal or human comprising administering to an animal or human subject an effective amount of 3,3',5,5'-tetraisopropyl-4,4'-biphenol and a pharmaceutically acceptable salt, ester, or solvate thereof, wherein the structure of 3,3',5,5'-tetraisopropyl-4,4'-biphenol is as shown in formula (I):

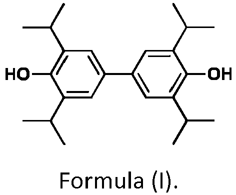

9. The method according to claim 8, wherein the ester is a monoester or diester of 3,3',5,5'-tetraisopropyl-4,4'-biphenol, and preferably the ester is a monoethyl ester represented by formula (II) or a diethyl ester represented by formula (III):

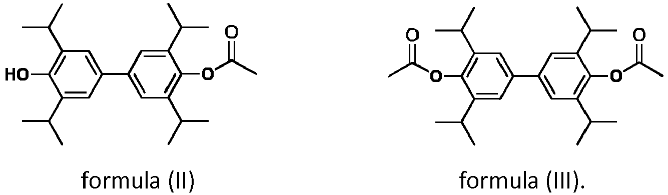

10. The method according to claim 8 or 9, wherein the pharmaceutically acceptable salt is a salt formed by 3,3',5,5'-tetraisopropyl-4,4'-biphenol with organic acid, inorganic acid or alkali metal, and wherein the pharmaceutically acceptable salts is, for example, sulfate, phosphate, hydrochloride, hydrobromide, acetate, oxalate, citrate, succinate, gluconate, tartrate, p-toluenesulfonate, benzenesulfonate, methanesulfonate, benzoate, lactate, maleate, lithium salt, sodium salt, potassium salt, or calcium salt.

11. The method according to claim 8 or 9, wherein 3,3',5,5'-tetraisopropyl-4,4'-biphenol and a pharmaceutically acceptable salt, ester, or solvate thereof can be formulated into a pharmaceutical composition which comprises the 3,3',5,5'-tetraisopropyl-4,4'-biphenol or a pharmaceutically acceptable salt, ester, or solvate thereof, and a pharmaceutical excipient.

12. The method according to claim 8 or 9, wherein, 3,3',5,5'-tetraisopropyl-4,4'-biphenol and a pharmaceutically acceptable salt, ester, or solvate thereof is formulated into tablet, capsule, injection, emulsion, liposome, lyophilized powder or microsphere formulation containing it, and
wherein the capsule is, for example, a soft capsule.

13. The method according to claim 8 or 9, wherein the treatment and/or prevention of ischemic stroke is achieved by improving cerebral ischemia and/or reperfusion neurological impairment; reducing cerebral ischemia and/or reperfusion cerebral infarction volume; reducing the endogenous oxygen free radical scavenger SOD consumption in brain tissues, reducing lipid peroxidation damage, while reducing serum MDA content; down-regulating cellular Fas expression in brain tissues; inhibiting brain cell apoptosis; and/or down-regulating cellular IL-1β and TNF-α expression in brain tissues.

14. The method according to claim 8 or 9, wherein the ischemic stroke includes damage caused by one or more of the following conditions: cerebral thrombosis, transient ischemic attack, basal ganglia infarction, atherosclerotic thrombotic cerebral infarction, lacunar cerebral infarction, cerebral embolism, and cerebrovascular dementia.

15. A process for the preparation of 3,3',5,5'-tetraisopropyl-4,4'-biphenol for the use according to any one of claims 1-7 or in the method according to any of claims 8-14, which comprises the step of preparing 3,3',5,5'-tetraisopropyl-4,4'-biphenol from a compound represented by the formula (IV)

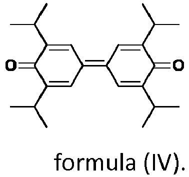

16. The process according to claim 15, wherein 3,3',5,5'-tetraisopropyl-4,4'-biphenol is prepared by dissolving the compound represented by the formula (IV) in an organic solvent, preferably ethyl acetate, and then reacting it with an aqueous solution containing sodium hydrosulfite, and wherein the aqueous solution containing sodium hydrosulfite is preferably an aqueous solution formed of sodium hydrosulfite and sodium hydroxide.

17. The process according to claim 15 or 16, wherein the compound represented by the formula (IV) is obtained by dissolving propofol in an organic solvent, preferably ethyl acetate, and then adding an inorganic salt thereto, and wherein the inorganic salt is preferably silver carbonate and anhydrous magnesium sulfate.

18. The process according to claim 15 including the steps of:
dissolving propofol in an organic solvent, preferably ethyl acetate, adding an inorganic salt, preferably silver carbonate and anhydrous magnesium sulfate thereto, and then stirring at room temperature;
after the reaction is complete, adding water to the reaction solution, filtering and washing the solid, removing the aqueous phase, drying and filtering the ethyl acetate phase, and evaporating the filtrate to dryness, followed by optional washing, to give rosy red crystal;
dissolving the rosy red crystal in ethyl acetate and mixing it with a sodium hydrosulfite solution, preferably, a sodium hydrosulfite solution of an aqueous solution of sodium hydrosulfite and NaOH, with optional stirring; then seperating the ethyl acetate phase and extracting the aqueous phase with ethyl acetate, followed by drying and filtering, evaporating the filtrate to dryness to give a light yellow solid, and then washing the light yellow solid to obtain 3,3',5,5'-tetraisopropyl-4,4'-biphenol as a white solid.

19. A process for the preparation of the monoethyl ester of formula (II) for the use according to any one of claims 2-7 or in the method according to any of claims 9-14, comprising the steps of:
dissolving 4'-benzyloxy-3,3',5,5'-tetraisopropylbiphenyl-4-acetate in an organic solvent, preferably methanol, at room temperature, and further adding a catalyst, preferably palladium-carbon thereto, evacuating, and charging hydrogen; after sealing, conducting a reaction at room temperature, filtering, and evaporating the filtrate to dryness to obtain 4'-hydroxy-3,3',5,5'-tetraisopropylbiphenyl-4-acetate as a white solid.

20. A process for the preparation of the diethyl ester of formula (II) for the use according to any one of claims 2-7 or in the method according to any of claims 9-14, comprising the steps of:
Adding 4,4'-dihydroxy-3,3',5,5'-tetraisopropylbiphenyl to acetic anhydride, and conducting a reaction at reflux under nitrogen, cooling the reaction solution to room temperature to remove acetic anhydride, adding water to the residue to give a white solid, and washing and drying the white solid to obtain 3,3',5,5'-tetraisopropylbiphenyl-4',4-diacetate.
